# EUROPEAN PATENT SPECIFICATION

(11) **EP 1 429 702 B1**
(45) Date of publication and mention of the grant of the patent: **23.03.2005**
(21) Application number: 02762569.8
(22) Date of filing: 26.09.2002
(51) Int. Cl.: A61F 13/15, A61L 15/42, A61L 15/44, A61L 15/62

(54) **THERAPEUTIC WOUND DRESSINGS WITH EXUDATE-DEPENDENT ENLARGEMENT OF APERTURES**
THERAPEUTISCHE WUNDPFLASTER MIT EXSUDAT-ABHÄNGIGER VERGRÖSSERUNG VON ÖFFNUNGEN
PANSEMENTS THERAPEUTIQUES AVEC AGRANDISSEMENT D'OUVERTURES EN FONCTION DE L'EXSUDAT

(30) Priority: 27.09.2001 GB 0123280
(43) Date of publication of application: 23.06.2004
(73) Proprietor: Johnson & Johnson Medical Ltd., Edinburgh EH2 4NH (GB)
(72) Inventor: KIRKWOOD, Andrew James, Cottingham, Hull HU16 4QG (GB); CULLEN, Breda Mary, Skipton, North Yorkshire BD23 1HR (GB); SILCOCK, Derek Walter, Skipton, North Yorkshire BD23 1QP (GB); WARRICK, Jonathon, Crosshills, North Yorkshire BD20 7TA (GB)
(74) Representative: James, Anthony Christopher W.P.
(86) International application number: PCT/GB2002/004361
(87) International publication number: WO 2003/026544

(56) References cited:
- EP-A- 0 875 222
- WO-A-02/47737
- US-A- 4 541 426
- US-A- 6 160 200

## Description

The present invention relates to wound dressings that can provide controlled delivery of one or more therapeutic agents to a wound.

The amount and composition of wound fluid (exudate) produced by a wound depends on the type of wound and the history of wound healing. For example, surgical wounds have an acute inflammatory phase of a few days during which discharge is significant, after which the rate of exudate production can be expected to fall sharply. Chronic wounds, such as ulcers, produce wound fluid containing elevated levels of protease enzymes. Infected wounds generally produce substantially more exudate than non-infected wounds, and the composition of the wound fluid is different. Burns produce large amounts of wound exudate having characteristic properties.

Infection of wounds by bacteria delays the healing process, since bacteria compete for nutrients and oxygen with macrophages and fibroblasts, whose activities are essential for the healing of the wound. Infection results when bacteria achieve dominance over the systemic and local factors of host resistance. Infection is therefore a manifestation of a disturbed host/bacteria equilibrium in favour of the invading bacteria. This elicits a systemic septic response, and also inhibits the multiple processes involved in wound healing. Lastly, infection can result in a prolonged inflammatory phase and thus slow healing, or may cause further necrosis of the wound. The granulation phase of the healing process will begin only after the infection has subsided.

Chronically contaminated wounds all contain tissue bacterial flora. These bacteria may be indigenous to the patient or might be exogenous to the wound. Closure, or eventual healing of the wound is often based on a physician's ability to control the level of the bacterial flora.

If clinicians could respond to wound infection as early as possible the infection could be treated topically as opposed to having to use antibiotics. This would also lead to less clinical intervention/hospitalisation and would reduce the use of antibiotics and other complications of infection.

Current methods used to identify bacterial infection rely mainly on judgement of the odour and appearance of a wound. With experience, it is possible to identify an infection in a wound by certain chemical signs such as redness or pain. Some clinicians take swabs that are then cultured in the laboratory to identify specific organisms, but this technique takes time.

Pain is also associated with infected and chronic wounds. Biochemically, pain is experienced when there is an increase of kinins (bradykinin) in the area of the wound. Kinins are produced by the proteolytic breakdown of kininogen, and the protease responsible for this is kallikrein. Kallikrein also stimulates the production of tissue plasminogen activator (t-PA)

It is also known to provide antimicrobial wound dressings. For example, such dressings are known having a liquid permeable wound contacting layer, an intermediate absorbent layer and an outer, liquid-impervious backing layer, in which one or more of the layers contains an antimicrobial agent. For example, EP-A-0599589 describes layered wound dressings having a wound contacting layer of a macromolecular hydrocolloid, an absorbent layer, and a continuous, microporous sheet intermediate the wound contacting layer and the absorbent layer. The absorbent layer contains a low molecular weight antimicrobial agent that can diffuse into the wound.

US-A-4541426 discloses a wound dressing comprising a conformable apertured film consisting of two layers laminated together, the first being the lesion-contacting layer formed from a material swellable on contact with water, the other either being non-swellable or swells less than the first layer. When in contact with a wet lesion, the different swell characteristics cause the apertures to open to allow passage of water. Medicaments (antibacterials) can be dispersed in the first layer. The more water passes into the first layer, the more medicament is released. The main technical problem solved is to keep the wound at a constant moisture level.

Previous therapeutic (e.g. antimicrobial) wound dressings suffer from the drawback that the release of the therapeutic agent is relatively unresponsive to the condition of the wound being treated. This is undesirable because all unnecessary medication can interfere with the processes of wound healing. In the case of antimicrobial wound dressings, unnecessary medication can result in resistant microorganisms.

There is thus a need for a wound treatment device that will selectively release therapeutic agents such as antimicrobial agents and/or pain relieving agents into wounds only when there is a clinical need. Such a device could provide early intervention with suitable treatment (e.g. a topical antimicrobial treatment) before severe clinical symptoms or wound chronicity sets in.

The present invention provides a wound dressing comprising: a wound contacting sheet having a wound facing surface and a back surface opposite the wound facing surface, and having apertures therein that open or enlarge in the presence of wound exudate; and a plurality of particles comprising one or more therapeutic agents located behind the back surface of the back sheet, wherein the particles are able to pass through the apertures to the wound facing surface of the sheet when the apertures are opened or enlarged in the presence of wound exudate.

The apertured sheet is provided with apertures that open or enlarge in the presence of wound exudate. This provides for increased liquid permeability through the sheet at higher exudation rates, thereby avoiding build-up of fluid under the dressing. The enlarged or opened apertures also allow the therapeutic particles to pass through the sheet to treat the wound selectively when wound exudation rates are sufficiently high to open or enlarge the apertures.

For example, the apertured sheet may be a laminate comprising a sheet of water swellable material laminated to a less water-swellable layer. Slits or other suitably shaped apertures are cut in the laminate. In use, differential swelling of the apertured layers in the presence of wound exudate causes the apertures to open, thereby increasing the permeability of the apertured sheet to wound fluid. Laminates of this type are described in EP-A-0122085.

In other embodiments, the apertured sheet has apertures that are at least partially blocked by a barrier material that breaks down in wound fluid to open the apertures. The breakdown of the barrier material may be by dissolution, or by enzymatic or other chemical degradation by the ingredients of wound fluid.

In these embodiments, the apertured sheet may be any medically acceptable wound contacting sheet, including woven and nonwoven textile materials. Preferably, the apertured sheet comprises a perforated thermoplastic film. Suitable thermoplastics include polyolefins such as polyethylene, copolymers such as ethylene methyl acrylate, or fluoropolymers such as polyvinylidene fluoride.

Typically, the apertures make up from 0.1% to 50% of the area of the sheet, more typically from 1% to 30% of the area of the apertured sheet, and preferably from 10% to 20% of the area of the apertured sheet.

Typically, the apertured sheet has from 1 to 30 apertures per square cm, for example from 4 to 15 apertures per square cm or from 5 to 10 apertures per square cm. In certain embodiments the apertures are uniformly distributed over the surface of the sheet, preferably in a regular pattern.

The mean area of each aperture may for example be from 0.01 to 10 mm², preferably from 0.1 to 4 mm², and more preferably about 1 mm².

In certain embodiments, the apertures before swelling have a ratio of maximum length to maximum width of from 1 to 10, preferably from 1 to 3, and more preferably from 1 to 1.5. Suitable aperture shapes include round, oval or regular polygonal

For example, the barrier material may comprise a water soluble material, such as a water soluble macromolecule. At medium to high levels of exudate the soluble material is dissolved by the exudate, thus revealing and restoring the full size of the apertures. At low levels of exudate or where there is a dry wound the soluble material will stay in place so that the perforations in the dressing remain occluded. This helps to maintain a moist wound healing environment.

Suitable soluble materials for partially or completely occluding the apertures include water soluble macromolecular materials (hydrogels) such as sodium alginate, sodium hyaluronate, alginate derivatives such as the propylene glycol alginate described in EP-A-0613692, and soluble hydropolymers formed from vinyl alcohols, vinyl esters, vinyl ethers and carboxy vinyl monomers, meth(acrylic) acid, acrylamide, N-vinyl pyrrolidone, acylamidopropane sulphonic acid, PLURONIC (Registered Trade Mark) (block polyethylene glycol, block polypropylene glycol) polystyrene-, maleic acid, NN-dimethylacrylamide diacetone acrylamide, acryloyl morpholine, and mixtures thereof. Suitable hydrogels are also described in US-A-5352508.

The soluble hydrogel composition may comprise at least 50% w/w based on the weight of the composition of the gel-forming macromolecular materials, more preferably at least 75% w/w. The soluble hydrocolloid can function as a liquid reservoir and humectant to help create a moist wound healing environment, and to assist this the soluble hydrogel composition may further comprise from 5 to 50% by weight, preferably from 15 to 40% by weight, on the same basis of one or more humectants such as glycerol. The soluble hydrogel composition may further contain up to 30% w/w, more preferably up to 15% w/w on the same basis of water.

The soluble hydrogel composition may further comprise up to 10% w/w, preferably from 0.1% to 5% w/w of a medicament based on the weight of the composition before swelling. Suitable medicaments include antiseptics such as silver sulfadiazine, chlorhexidine, triclosan or povidone iodine, analgesics, steroids, antibiotics, growth factors or mixtures thereof.

Other suitable materials for partially or completely occluding the apertures of the sheet are polymeric materials that are not soluble in water, but that are bioerodible in wound fluid. Examples include polylactide/polyglycolide copolymers, oxidized regenerated cellulose, and mixtures thereof.

Other suitable materials for partially or completely occluding the apertures of the sheet are pH-sensitive materials that are substantially insoluble in water at 25°C under acidic conditions, but substantially soluble in water at 25°C under neutral or alkaline conditions. Whilst it is no simple matter to determine the actual pH at a wound site, it appears that the pH of chronic or infected wounds is neutral or slightly alkaline, whereas the pH of intact skin is slightly acidic (pH 4 or 5).

Preferably, the pH-sensitive material is substantially insoluble in water at 25°C and pH 4 and substantially soluble in water at 25°C and pH 8. Preferably, the polymer becomes soluble with increasing pH at a pH in the range of 5 to 7, more preferably 5.5 to 6.5. In this context the term "soluble" preferably denotes an equilibrium solubility of the material greater than 1%w/w in water at 25°C. Particularly suitable are film-forming polymers and mixtures, such as those used to provide enteric coatings on orally administered medicaments. The pH-sensitive material may be associated with a buffer, such as an acid buffer, to render it insoluble until a predetermined amount of neutral or alkaline exudate has formed.

Preferably, the pH-sensitive material comprises a polymer selected from the group consisting of cellulose derivatives, starch derivatives, pectins, polyacrylates, polyvinyl acetate phthalate, and mixtures thereof.

Preferred cellulose derivatives are selected from cellulose acetate phthalate, cellulose acetate trimellitate, hydroxypropyl methyl cellulose, hydroxypropyl methyl cellulose acetate succinate, carboxymethyl ethyl cellulose, oxidised regenerated cellulose, and mixtures thereof.

Preferred polyacrylates are selected from the copolymers of methacrylic acid with methyl methacrylate. Particularly preferred are various copolymers of this type sold under the Registered Trade Mark EUDRAGIT. By varying the ratio of methacrylic acid to methyl methacrylate it is possible to control the pH at which these copolymers dissolve in order to optimise the properties of the material.

Other suitable barrier materials for partially or completely occluding the apertures of the sheet comprise substances that are substantially insoluble in water at 25°C under neutral conditions, but that are broken down by the action of one or more active substances present in wound fluid. Preferably, the substances are substrates for one or more enzymes present in wound fluid, for example protease enzymes or lysozome. It has been discovered that wound fluid from infected wounds, and from wounds that are apparently not clinically infected but which go on to become infected within a few days, have high levels of neutrophil elastase activity and may also have high levels of other inflammatory enzymes, such as macrophage proteases, other neutrophil proteases, bacterial collagenase, plasmin, hyaluronidase, kallikrein or t-PA. Accordingly, the degradable substances preferably are substrates for a protease selected from the group consisting of elastase, collagenase, pectinase, matrix metalloproteinases, and mixtures thereof. Preferred substrate materials include substances selected from the group consisting of elastin, fibronectin, collagen, crosslinked gelatin, fibrinogen, casein, hyaluronic acid, plasminogen, fibrin, and mixtures thereof.

The barrier material preferably comprises at least 25% of the soluble macromolecule, pH-sensitive material or degradable substance, more preferably at least 50% w/w based on the dry weight of the material. The barrier material may further comprise preferably from 5 to 50% by weight, preferably from 15 to 40% by weight, on the same basis of one or more humectants such as glycerol or sorbitol, and optionally 5 to 50% by weight, preferably from 15 to 40% by weight, on the same basis of one or more plasticisers such as polyethylene glycol.

In yet other embodiments of the present invention, the apertured sheet is formed from a water-swellable hydrogel composition, whereby swelling of the hydrogel on exposure to wound fluid causes the apertures to enlarge.

In these embodiments, the apertures typically make up from 0.1% to 50% of the area of the sheet before swelling, more typically from 1% to 30% of the area of the sheet before swelling, and preferably from 10% to 20% of the area of the sheet before swelling.

The apertured hydrogel sheet enables a moist wound environment to be maintained for prolonged periods, over a wide range of wound exudation rates. When the exudation rate is high, the apertured sheet expands and the resulting increase in the size of the apertures increases their liquid permeablility and allows the particles to pass through the sheet. Furthermore, the hydrogel absorbs moisture vapor and functions as a humectant to preserve a moist wound contacting surface.

Preferably, the area of the apertures is increased by at least 25%, for example at least 50% by swelling the sheet in water at 25ºC for 60 minutes.

Typically, the water-swellable hydrogel sheet has from 1 to 30 apertures per square cm, for example from 4 to 15 apertures per square cm or from 5 to 10 apertures per square cm. In certain embodiments the apertures are uniformly distributed over the surface of the sheet, preferably in a regular pattern.

The apertures are preferably small, both in order to retain the particles behind the sheet and to provide the greatest proportional increase in liquid permeability when the hydrogel sheet swells. For example, the mean area of each aperture may be from 0.01 to 10 mm², preferably from 0.1 to 4 mm², and more preferably about 1 mm².

The apertures may have any suitable shape, as long as the size of the apertures increases when the hydrogel swells so as to increase the liquid permeability. In certain embodiments, the apertures before swelling have a ratio of maximum length to maximum width of from 1 to 10, preferably from 1 to 3, and more preferably from 1 to 1.5. Suitable aperture shapes include round, oval or regular polygonal

The apertured hydrogel sheet is preferably self-supporting. That is to say, the apertured sheet preferably does not have any reinforcing or supporting sheet, web, net or member embedded therein or laminated thereto. The apertured hydrogel sheet normally consists essentially of the hydrogel composition as hereinafter described. Naturally, the self-supporting nature of the sheet implies that the hydrogel composition has a minimum inherent strength. Preferably, the hydrogel composition (measured on a continuous strip (2.5 cm wide) of the hydrogel in accordance with ASTM 412) has a breaking force of 0.5- 10 N, more preferably 1-5 N.

The term "water-swellable hydrogel composition" refers to compositions that absorb water to form a gel with water under physiological conditions of temperature and pH. Such compositions comprise medically acceptable macromolecular materials that have the ability to swell and absorb wound fluid while maintaining a strong integral structure. Normally, the hydrogel composition is substantially insoluble in water under physiological conditions, whereby the hydrogel is not washed away by the wound fluid. The hydrogel may comprise a biopolymer, i.e it may be formed from a polymer found in nature such as collagen, gelatin or alginate. The hydrogel may be bioabsorbable. That is to say, it may undergo gradual resorption *in vivo.*

Typically, the water-swellable hydrogel according to these embodiments absorbs at least 10% w/w of water preferably at least 25% w/w of water, more preferably at least 50% w/w of water, and still more preferably at least 100% w/w of water on immersion at 25ºC for 60 minutes, based on the weight of the sheet before immersion.

Exemplary insoluble gels include certain cross-linked polyacrylate gels such as those described in EP-A-0676457, calcium alginate gels, cross-linked hyaluronate gels, gels of alginate derivatives such as propylene glycol alginate, and gels wherein the hydropolymer is formed from vinyl alcohols, vinyl esters, vinyl ethers and carboxy vinyl monomers, meth(acrylic) acid, acrylamide, N-vinyl pyrrolidone, acylamidopropane sulphonic acid, PLURONIC (Registered Trade Mark) (block polyethylene glycol, block polypropylene glycol) polystyrene-, maleic acid, NN-dimethylacrylamide diacetone acrylamide, acryloyl morpholine, and mixtures thereof. Suitable hydrogels are also described in US-A-5352508.

Preferably, the water-swellable hydrogel composition comprises a macromolecular material selected from polyurethane gels, biopolymer gels, carboxymethyl cellulose gels, hydroxyethyl cellulose gels, hydroxy propyl methyl cellulose, polyacrylate and mixtures thereof. Suitable biopolymer gels include alginates, pectins, gelatin gels, galactomannans such as guar and xanthan, chitosan, gelatin, hyaluronates and mixtures thereof. Some of these biopolymer materials also promote wound healing.

Preferably, the gels are chemically or physically cross-linked, and the chemical cross-linking may be either covalent or ionic.

The water-swellable hydropolymer composition may comprise at least 50% w/w based on the weight of the sheet before swelling of the gel-forming macromolecular materials, more preferably at least 75% w/w. The hydrogel material may further comprise from 5 to 50% by weight, preferably from 15 to 40% by weight, on the same basis of one or more humectants such as glycerol. The hydrogel material may further contain up to 30% w/w, more preferably up to 15% w/w on the same basis of water.

The therapeutic particles are released into the wound through the apertured sheet after the apertures have opened or enlarged, but preferably not before. It follows that the particles are shaped, sized and distributed such that they can pass through the opened or enlarged apertures, but preferably do not pass through the apertures before opening or enlargement. The particles normally do not dissolve directly in wound fluid, although they may undergo gradual breakdown by hydrolysis or similar biodegradative reactions.

Preferably, the particles have a mesh size such that at least 90% of the particles are retained by a mesh (U.S. Sieve size) of from 10 to 200, and more preferably from 15 to 50. This corresponds to a minimum effective diameter of from about 0.075mm to about 2mm, preferably from about 0.3mm to about 1.4mm. Preferably, at least 90% of the particles pass a mesh of about 5, and more preferably at least 90% of the particles pass a mesh of about 10. Preferably, at least 50 wt.% of the particles have an aspect ratio (i.e. ratio of the largest dimension to the smallest dimension) of from 1 to 3. Especially suitable are substantially spherical particles. Preferably, at least 50 wt.% of the particles have a maximum dimension in the range of from about 0.05mm to about 1mm.

Preferably, the particles provide sustained release of the therapeutic agent in wound fluid. For example, the particles may comprise a bioerodible substance having the therapeutic agents dispersed or encapsulated therein. Suitable bioerodible substances include proteins such as albumin, collagen, cross-linked gelatin or zein, polysaccharides such as oxidized regenerated cellulose, biodegradable synthetic polymers such as polylactate/polyglycolate copolymers, glycosaminoglycans such as hyaluronate, and mixtures thereof. It is also envisaged that the particles could be liposomes.

The particles may be made by any suitable technique, including comminution, coacervation, or two-phase systems for example as described in US-A-3886084. Techniques for the preparation of medicated microspheres for drug delivery are reviewed, for example, in Polymeric Nanoparticles and Microspheres, Guiot and Couvreur eds., CRC Press (1986).

A preferred method for preparation of the microparticles is coacervation, which is especially suited to the formation of particles in the preferred size range of 100 to 500 micrometers having a high loading of therapeutic agents. Coacervation is the term applied to the ability of a number of aqueous solutions of colloids, to separate into two liquid layers, one rich in colloid solute and the other poor in colloid solute. Factors which influence this liquid-liquid phase separation are: (a) the colloid concentration, (b) the solvent of the system, (c) the temperature, (d) the addition of another polyelectrolyte, and (e) the addition of a simple electrolyte to the solution.

Coacervation can be of two general types. The first is called "simple" or "salt" coacervation where liquid phase separation occurs by the addition of a simple electrolyte to a colloidal solution. The second is termed "complex" coacervation where phase separation occurs by the addition of a second colloidal species to a first colloidal solution, the particles of the two dispersed colloids being oppositely charged. Generally, materials capable of exhibiting an electric charge in solution (i.e. materials which possess an ionizable group) are coacervable. Such materials include natural and synthetic macromolecular species such as gelatin, acacia, tragacanth, styrene-maleic anhydride copolymers, methyl vinyl ether-maleic anhydride copolymers, polymethacrylic acid, and the like.

If, prior to the initiation of coacervation, a water-immiscible material, such as an oil, is dispersed as minute droplets in an aqueous solution or sol or an encapsulating colloidal material, and then, a simple electrolyte, such as sodium sulfate, or another, oppositely charged colloidal species is added to induce coacervation, the encapsulating colloidal material forms around each oil droplet, thus investing each of said droplets in a liquid coating of the coacervated colloid. The liquid coatings which surround the oil droplets must thereafter be hardened by cross-linking to produce solid-walled microcapsules

The one or more therapeutic agents may be any substance suitable for the treatment of wounds. In certain embodiments the therapeutic agents are selected from the group consisting of antiseptics, antibiotics, analgesics, steroids and growth factors. Preferred therapeutic agents are the antimicrobials, in particular antibiotics and antiseptics such as colloidal silver, silver sulfadiazine, povidone iodine, chlorhexidine, and mixtures thereof. The microparticles are preferably loaded with from 1 to 90 wt.%, more preferably from 3 to 50 wt.% of the therapeutic agents.

The microspheres may for example be provided in a layer behind the apertured sheet, for example by coating them onto the back of the apertured sheet in a suitable water-soluble matrix, or they may be coated onto the surface of an absorbent layer behind the apertured sheet, as described further below.

Preferably, the density of the microspheres is from 1 to 1000 g/m², preferably from 10 to 100g/m² of the apertured sheet.

Preferably, the wound dressing according to the present invention further comprises an absorbent layer and/or a backing layer in addition to the apertured sheet and the microspheres, in which case the apertured sheet is preferably the wound-facing top sheet of the dressing.

The area of the optional absorbent layer is typically in the range of from 1cm² to 200cm², more preferably from 4cm² to 100cm².

The optional absorbent layer may be any of the layers conventionally used for absorbing wound fluids, serum or blood in the wound healing art, including gauzes, nonwoven fabrics, superabsorbents, hydrogels and mixtures thereof. Preferably, the absorbent layer comprises a layer of absorbent foam, such as an open celled hydrophilic polyurethane foam prepared in accordance with EP-A-0541391. In other embodiments, the absorbent layer may be a nonwoven fibrous web, for example a carded web of viscose staple fibers. The basis weight of the absorbent layer may be in the range of 50-500g/m², such as 100-400g/m². The uncompressed thickness of the absorbent layer may be in the range of from 0.5mm to 10mm, such as 1 mm to 4mm. The free (uncompressed) liquid absorbency measured for physiological saline may be in the range of 5 to 30 g/g at 25°C.

Preferably, the dressing further comprises a backing layer over the back face of the apertured sheet. The backing layer preferably provides a barrier to passage of microorganisms through the dressing and further preferably blocks the escape of wound fluid from the dressing. The backing layer may extend beyond at least one edge of the absorbent layer to provide an adhesive-coated margin adjacent to the said edge for adhering the dressing to a surface, such as to the skin of a patient adjacent to the wound being treated. An adhesive-coated margin may extend around all sides of the absorbent layer, so that the dressing is a so-called island dressing. However, it is not necessary for there to be any adhesive-coated margin.

Preferably, the backing layer is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-cell. A suitable backing sheet material is the polyurethane film available under the Registered Trade Mark ESTANE 5714F.

The adhesive (where present) layer should be moisture vapor transmitting and/or patterned to allow passage of water vapor therethrough. The adhesive layer is preferably a continuous moisture vapor transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are preferred.

Preferably, the adhesive layer extends outwardly from the absorbent layer and the envelope to form an adhesive-coated margin on the backing sheet around the adhesive layer as in a conventional island dressing.

Preferably, the wound dressing according to the present invention is sterile and packaged in a microorganism-impermeable container.

An embodiment of the present invention will now be described further, by way of example, with reference to the accompanying drawings, in which:
Figure 1 shows a perspective view of the lower (wound contacting) surface of a wound dressing according to the invention with the wound contacting sheet partially cut away; and

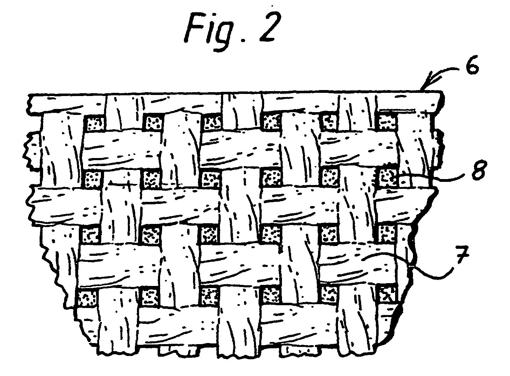

Figure 2 shows a plan view of a portion of the wound contacting sheet from the dressing of Fig. 1.

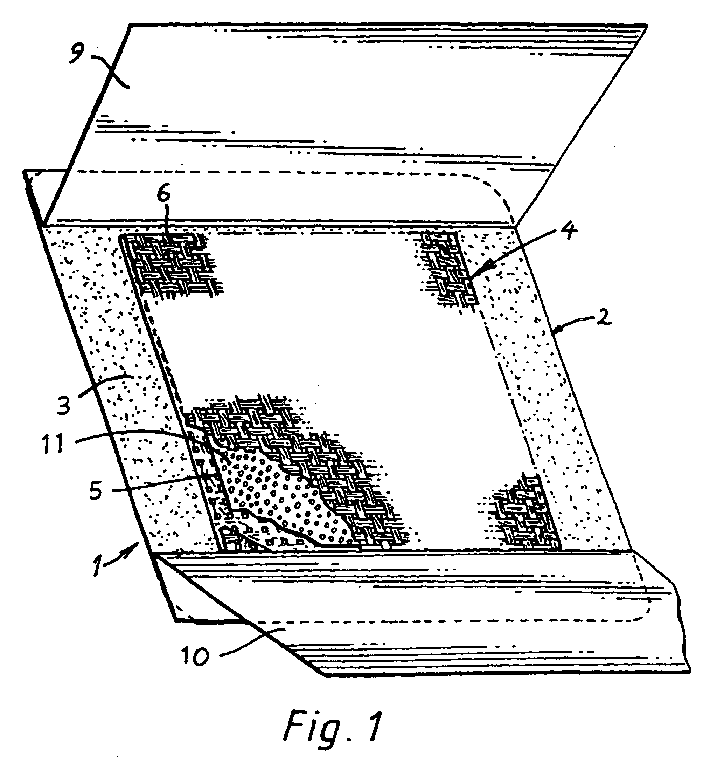

Referring to Figure 1, the wound dressing 1 is an island-type self-adhesive wound dressing comprising a backing layer 2 of microporous liquid-impermeable polyurethane foam, such as ESTANE 5714F (Registered Trade Mark). The backing layer is permeable to water vapor, but impermeable to wound exudate and microorganisms.

The backing layer 2 is coated with a substantially continuous layer 3 of pressure-sensitive polyurethane adhesive. An absorbent island 4 is adhered to a central region of the adhesive-coated backing sheet 2.

The absorbent island 4 comprises an absorbent layer 5 of hydrophilic polyurethane foam prepared as described in EP-A-0541391 and having a basis weight of about 350g/m² and a thickness of about 1.5 mm.

A wound contacting sheet 6 extends over the absorbent layer 5 and is wrapped around the absorbent layer 5, and adhered to the backing layer 2 behind the absorbent layer 5 by the adhesive 3. The wound contacting sheet 6 consists of a support layer 7 of vacuum mesh-perforated ethylene methyl acrylate (EMA) film in which the apertures 8 are occluded by a sodium alginate water-soluble hydrogel. The apertures have a mean non-occluded diameter of about 0.4mm and a density of about 25 apertures per square cm.

Intermediate the wound contacting sheet 6 and the absorbent layer 5 there is provided a layer 11 of cross-linked gelatin microspheres having a mesh size of -18 (+40), i.e. an effective minimum diameter in the range of about 0.4mm to 1 mm, and containing 3%w/w of chlorhexidine antiseptic. The microspheres are coated onto the surface of the absorbent layer as a dispersion in soluble sodium alginate gel at a dry microsphere density of 10g/m².

The wound facing surface of the dressing shown in Figure 1 is protected by two silicone-coated release papers 9,10. The dressing is packaged in a microorganism-impermeable pouch (not shown), and sterilised using gamma radiation.

In use, the dressing 1 is removed from the package, the release papers 9,10 are removed, and the dressing is adhered to the skin around the wound by the adhesive layer 3, with the wound contacting sheet in contact with the wound to provide a sterile and absorbent dressing. At low wound exudate levels, the hydrogel absorbs wound exudate and maintains a moist environment at the wound surface. As more exudate is produced, the hydrogel dissolves, allowing the excess exudate to escape through the perforated sheet 7 into the absorbent layer 5. In this way, the dressing can provide an improved wound healing environment for an extended time on both high- and low- exuding wounds. Furthermore, the dissolution of the hydrogel triggers the release of antimicrobial particles through the apertured sheet into the wound in response to increased exudate production by infected wounds, thereby providing sustained, selective and targeted release of the antiseptic.

The above embodiment has been described by way of example only. Many other embodiments falling within the scope of the accompanying claims will be apparent to the skilled reader.

## Claims

1. A wound dressing comprising:
a wound contacting sheet having a wound facing surface and a back surface opposite the wound facing surface, and having apertures therein that open or enlarge in the presence of wound exudate; and
a plurality of particles comprising one or more therapeutic agents located behind the back surface of the wound contacting sheet, wherein the particles are able to pass through the apertures to the wound facing surface of the sheet when the apertures are opened or enlarged in the presence of said wound exudate.

2. A wound dressing according to claim 1, wherein the wound contacting sheet is an apertured laminate comprising a layer of water swellable material laminated to a less water-swellable layer, whereby differential swelling of the apertured layers in the presence of wound exudate causes the apertures to open.

3. A wound dressing according to claim 1, wherein the wound contacting sheet comprises an apertured layer having apertures that are at least partially blocked by a material that breaks down in wound fluid to open the apertures.

4. A wound dressing according to claim 3, wherein the material that breaks down in wound fluid comprises a water-soluble macromolecule.

5. A wound dressing according to claim 3 or 4, wherein the material that breaks down in wound fluid comprises a pH-sensitive material that is substantially insoluble in water at 25°C under acidic conditions, but substantially soluble in water at 25°C under neutral or alkaline conditions.

6. A wound dressing according to claim 3 or 4, wherein the material that breaks down in wound fluid comprises a degradable material that is substantially insoluble in water at 25°C under neutral conditions, but that is broken down by the action of one or more active substances present in wound fluid.

7. A wound dressing according to claim 6, wherein the degradable material comprises a substance selected from the group consisting of elastin, fibronectin, collagen, crosslinked gelatin, fibrinogen, casein, hyaluronates, plasminogen, fibrin, oxidized cellulose, polylactide/polyglycolide copolymers, and mixtures thereof.

8. A wound dressing according to claim 1, wherein the wound contacting sheet is formed from an apertured layer of water-swellable hydrogel composition, whereby swelling of the hydrogel on exposure to wound fluid causes the apertures to enlarge.

9. A wound dressing according to any preceding claim, wherein the particles are retained by a mesh of mesh size from about 2mm (10 mesh) to about 0.075mm (200 mesh).

10. A wound dressing according to any preceding claim, wherein the particles have an aspect ratio of from 1 to 3.

11. A wound dressing according to claim 10, wherein the particles are substantially spherical.

12. A wound dressing according to any preceding claim, wherein the particles have a maximum dimension in the range of from 0.05mm to 1 mm.

13. A wound dressing according to any preceding claim, wherein the particles provide sustained release of the therapeutic agent in wound fluid.

14. A wound dressing according to any preceding claim, wherein the particles comprise a bioerodible substance having the therapeutic agents dispersed or encapsulated therein.

15. A wound dressing according to claim 14, wherein the bioerodible substance is selected from the group consisting of proteins, polysaccharides, biodegradable synthetic polymers, glycosaminoglycans, and mixtures thereof.

16. A wound dressing according to any preceding claim wherein the therapeutic agents are selected from the group consisting of antiseptics, antibiotics, analgesics, steroids and growth factors.

17. A wound dressing according to any preceding claim which is sterile and packaged in a microorganism-impermeable container.

## Patentansprüche

1. Wundverband, der Folgendes umfasst:
eine Wundkontaktschicht mit einer der Wunde zugewandten Oberfläche und einer Rückfläche gegenüber der der Wunde zugewandten Oberfläche und mit Öffnungen darin, die sich beim Vorhandensein von Wundexsudat öffnen oder vergrößern, und
eine Vielzahl von Partikeln, die ein oder mehrere therapeutische Mittel umfassen, die sich hinter der Rückfläche der Wundkontaktschicht befinden, **dadurch gekennzeichnet, daß**
die Partikel durch die Öffnungen zu der der Wunde zugewandten Oberfläche der Folie passieren können, wenn sich die Öffnungen beim Vorhandensein des Wundexsudats geöffnet oder vergrößert haben.

2. Wundverband nach Anspruch 1, **dadurch gekennzeichnet, daß**
die Wundkontaktschicht ein mit Öffnungen versehenes Laminat ist, das eine Lage aus mit Wasser quellfähigem Material umfasst, die an eine mit Wasser weniger quellfähige Lage laminiert ist, wobei ein unterschiedliches Quellen der mit Öffnungen versehenen Lagen beim Vorhandensein von Wundexsudat zum Öffnen der Öffnungen führt.

3. Wundverband nach Anspruch 1, **dadurch gekennzeichnet, daß**
die Wundkontaktschicht eine mit Öffnungen versehene Lage umfasst, die Öffnungen aufweist, die zumindest teilweise durch ein Material blockiert sind, das sich in Wundflüssigkeit zersetzt, um die Öffnungen zu öffnen.

4. Wundverband nach Anspruch 3, **dadurch gekennzeichnet, daß**
das Material, das sich in Wundflüssigkeit zersetzt, ein wasserlösliches Makromolekül umfasst.

5. Wundverband nach Anspruch 3 oder 4, **dadurch gekennzeichnet, daß**
das Material, das sich in Wundflüssigkeit zersetzt, ein pH-empfindliches Material umfasst, das bei 25°C unter sauren Bedingungen in Wasser im Wesentlichen nicht löslich, aber bei 25°C unter neutralen oder basischen Bedingungen in Wasser im Wesentlichen löslich ist.

6. Wundverband nach Anspruch 3 oder 4, **dadurch gekennzeichnet, daß**
das Material, das sich in Wundflüssigkeit zersetzt, ein abbaubares Material umfasst, das bei 25°C unter neutralen Bedingungen in Wasser im Wesentlichen nicht löslich ist, aber durch die Wirkung eines oder mehrerer in der Wundflüssigkeit vorhandener Wirkstoffe zersetzt wird.

7. Wundverband nach Anspruch 6, **dadurch gekennzeichnet, daß**
das abbaubare Material eine Substanz umfasst, die aus der aus Elastin, Fibronectin, Collagen, vernetzter Gelatine, Fibrinogen, Casein, Hyaluronaten, Plasminogen, Fibrin, oxidierter Zellulose, Polylactid/Polyglykolid-Copolymeren und Mischungen davon bestehenden Gruppe ausgewählt wird.

8. Wundverband nach Anspruch 1, **dadurch gekennzeichnet, daß**
die Wundkontaktschicht aus einer mit Öffnungen versehenen Lage aus einer mit Wasser quellfähigen Hydrogel-Zusammensetzung gebildet ist, wobei das Quellen des Hydrogels durch Kontakt mit Wundflüssigkeit eine Vergrößerung der Öffnungen verursacht.

9. Wundverband nach einem der vorhergehenden Ansprüche, **dadurch gekennzeichnet, daß**
die Partikel von einem Gewebe mit einer Maschenweite von etwa 2 mm (10 mesh) bis etwa 0,075 mm (200 mesh) zurückgehalten werden.

10. Wundverband nach einem der vorhergehenden Ansprüche, **dadurch gekennzeichnet, daß**
die Partikel ein Seitenverhältnis von 1 bis 3 aufweisen.

11. Wundverband nach Anspruch 10, **dadurch gekennzeichnet, daß**
die Partikel im Wesentlichen kugelförmig sind.

12. Wundverband nach einem der vorhergehenden Ansprüche, **dadurch gekennzeichnet, daß**
die Partikel eine maximale Abmessung im Bereich von 0,05 mm bis 1 mm aufweisen.

13. Wundverband nach einem der vorhergehenden Ansprüche, **dadurch gekennzeichnet, daß**
die Partikel für eine andauernde Freisetzung des therapeutischen Mittels in Wundflüssigkeit sorgen.

14. Wundverband nach einem der vorhergehenden Ansprüche, **dadurch gekennzeichnet, daß**
die Partikel eine bioerodierbare Substanz umfassen, in der die therapeutischen Mittel dispergiert oder eingekapselt sind.

15. Wundverband nach Anspruch 14, **dadurch gekennzeichnet, daß**
die bioerodierbare Substanz aus der aus Proteinen, Polysacchariden, biologisch abbaubaren synthetischen Polymeren, Glykosaminoglykanen und Mischungen davon bestehenden Gruppe ausgewählt wird.

16. Wundverband nach einem der vorhergehenden Ansprüche, **dadurch gekennzeichnet, daß**
die therapeutischen Mittel aus der aus Antiseptika, Antibiotika, Analgetika, Steroiden und Wachstumsfaktoren bestehenden Gruppe ausgewählt sind.

17. Wundverband nach einem der vorhergehenden Ansprüche, der steril und in einem Behälter verpackt ist, in den keine Mikroorganismen eindringen können.

## Revendications

1. Pansement pour plaie qui comprend :
une feuille de contact avec la plaie possédant une surface en regard de la plaie et une surface arrière opposée à la surface en regard de la plaie, et dans laquelle se trouvent des ouvertures qui s'ouvrent ou s'agrandissent en présence de l'exsudat de la plaie ; et
un grand nombre de particules comprenant un ou plusieurs agents thérapeutiques situées derrière la surface arrière de la feuille de contact avec la plaie, dans lequel les particules sont capables de traverser les ouvertures vers la surface en regard de la plaie de la feuille lorsque les ouvertures sont ouvertes ou agrandies en présence dudit exsudat de la plaie.

2. Pansement pour plaie selon la revendication 1, dans lequel la feuille de contact avec la plaie est un stratifié à ouvertures comprenant une couche de matière apte à gonfler dans l'eau stratifiée à une couche moins susceptible de gonfler dans l'eau, et ainsi le gonflement différentiel des couches à ouvertures en présence de l'exsudat de la plaie amène les ouvertures à s'ouvrir.

3. Pansement pour plaie selon la revendication 1, dans lequel la feuille de contact avec la plaie comprend une couche à ouvertures possédant des ouvertures qui sont au moins partiellement bloquées par un matériau qui se dégrade dans la substance liquide de la plaie pour ouvrir les ouvertures.

4. Pansement pour plaie selon la revendication 3, dans lequel le matériau qui se dégrade dans la substance liquide de la plaie comprend une macromolécule soluble dans l'eau.

5. Pansement pour plaie selon la revendication 3 ou 4, dans lequel le matériau qui se dégrade dans la substance liquide de la plaie comprend un matériau sensible au pH qui est pratiquement insoluble dans l'eau à 25°C dans des conditions acides, mais nettement soluble dans l'eau à 25°C dans des conditions neutres ou alcalines.

6. Pansement pour plaie selon la revendication 3 ou 4, dans lequel le matériau qui se dégrade dans la substance liquide de la plaie comprend un matériau dégradable qui est pratiquement insoluble dans l'eau à 25 °C dans des conditions neutres, mais qui se dégrade par l'action d'une ou plusieurs substances actives présentes dans la substance liquide de la plaie.

7. Pansement pour plaie selon la revendication 6, dans lequel le matériau dégradable comprend une substance choisie dans l'ensemble comprenant l'élastine, la fibronectine, le collagène, la gélatine réticulée, le fibrinogène, la caséine, les hyaluronates, le plasminogène, la fibrine, la cellulose oxydée, les copolymères polylactide/polyglycolide et leurs mélanges.

8. Pansement pour plaie selon la revendication 1, dans lequel la feuille de contact avec la plaie est formée à partir d'une couche à ouvertures d'une composition d'hydrogel gonflable dans l'eau, grâce à quoi le gonflement de l'hydrogel lors de l'exposition à la substance liquide de la plaie amène les ouvertures à s'agrandir.

9. Pansement pour plaie selon l'une quelconque des revendications précédentes , dans lequel les particules sont maintenues par un treillis d'une ouverture de maille d'environ 2 mm ( 10 mesh) à environ 0,075 mm ( 200 mesh ).

10. Pansement pour plaie selon l'une quelconque des revendications précédentes, dans lequel les particules ont un rapport dimensionnel de 1 à 3.

11. Pansement pour plaie selon la revendication 10, dans lequel les particules sont pratiquement sphériques.

12. Pansement pour plaie selon l'une quelconque des revendications précédentes, dans lequel les particules ont une dimension maximale comprise dans l'intervalle de 0,05 mm à 1 mm.

13. Pansement pour plaie selon l'une quelconque des revendications précédentes, dans lequel les particules offrent une libération prolongée de l'agent thérapeutique dans la substance liquide de la plaie.

14. Pansement pour plaie selon l'une quelconque des revendications précédentes, dans lequel les particules comprennent une substance bioérodible dans laquelle les agents thérapeutiques sont dispersés ou encapsulés.

15. Pansement pour plaie selon la revendication 14, dans lequel la substance bioéradible est choisie dans l'ensemble comprenant protéines, polysaccharides, polymères synthétiques biodégradables, glycosaminoglycanes et leurs mélanges.

16. Pansement pour plaie selon l'une quelconque des revendications précédentes, dans lequel les agents thérapeutiques sont choisis dans le groupe comprenant les antiseptiques, les antibiotiques, les analgésiques, les stéroïdes et les facteurs de croissance.

17. Pansement pour plaie selon l'une quelconque des revendications précédentes, qui est stérile et conditionné dans un emballage imperméable aux microorganismes.
